# EUROPEAN PATENT SPECIFICATION

(11) **EP 0 436 940 B1**
(45) Date of publication and mention of the grant of the patent: **14.05.2003**
(21) Application number: 90125656.0
(22) Date of filing: 28.12.1990
(51) Int. Cl.: C07D 493/04, D06P 3/54

(54) **Process for producing benzodi-furanone compounds useful for dyeing or printing hydrophobic fiber materials**
Verfahren zur Herstellung von Benzodifuranon-Verbindungen, geeignet zum Färben oder Bedrucken von hydrophobem Fasermaterial
Procédé pour la production de composés de benzodifuranones utilisables par coloration ou impression de matériau fibreux hydrophobique

(30) Priority: 12.01.1990 JP 522390
(43) Date of publication of application: 17.07.1991
(73) Proprietor: SUMITOMO CHEMICAL COMPANY LIMITED, Osaka-shi, Osaka 541-0041 (JP)
(72) Inventor: Sekihachi, Junichi, Tyonaka-shi, Osaka 561 (JP); Yamamoto, Jun, Minoo-shi, Osaka 562 (JP); Kayane, Yutaka, Ikoma-shi, Nara 630-01 (JP)
(74) Representative: VOSSIUS & PARTNER

(56) References cited:
- EP-A- 0 294 029
- EP-A- 0 305 886
- CHEMICAL ABSTRACTS, vol. 112, no. 22, May 28, 1990, Columbus, Ohio, USA SUMITOMO CHEMICAL CO "Heterocyclic dye and its use in dyeing or printing of hydrophobic textiles" page 107, column 2, abstract -No. 200 605u ; & JP-A-01 258 677

## Description

The present invention relates to a process for producing benzodifuranone compounds. More specifically, the present invention relates to a process for producing benzodifuranone compounds useful for dyeing or printing hydrophobic fiber materials, for example, polyester fibers or fiber materials containing the same, in a red color.

Various benzodifuranone compounds useful for dyeing or printing hydrophobic fiber materials and a method for preparing the same are mentioned in, for example, JP-A-61-54058, 1-258677 and 1-36859. In addition, a process using a mandelic acid compound as one of starting materials and a process using phenylglyoxylic acid compounds as one of starting materials are proposed (JP-A-60-178889 and EP-A-252 406).

However, these known processes all are not satisfactory yet from an economical point of view.

The object of the present invention is to find a process for commercially producing benzodifuranone compounds. As a result, the present invention is attained.

The present invention provides a process for producing benzodifuranone compounds of the following formula (I), 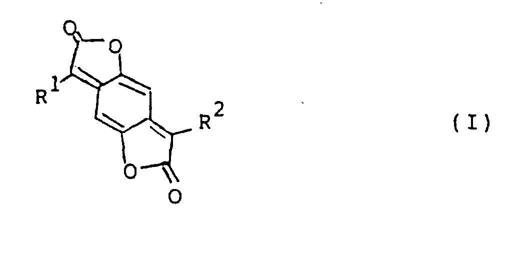 wherein R¹ and R² are each independently a naphthyl group or an unsubstituted or substituted phenyl group, which comprises allowing benzofuran compounds of the following formula (II), 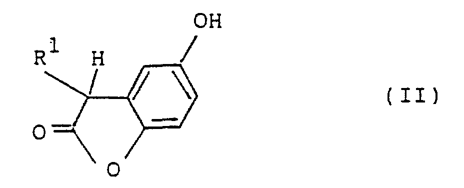 wherein R¹ is as defined above, to react at a temperature of 30° to 180°C in presence of acid catalysts with acetonitrile
compounds of the following formula (III), 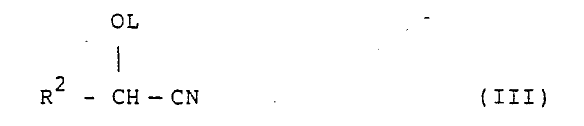 wherein R² is as defined above, L is -COR³, -CO₂R⁴ or -SO₂R⁵ in which R³, R⁴ and R⁵ are each independently an C₁₋₁₀-alkyl or a phenyl group which may be substituted, followed by oxidation. Preferred are alkyl groups having 2 to 4 carbon atoms. The substituents of the phenyl group are preferably C₁₋₄ alkyl groups, C₁₋₄

In the above formula (I), the phenyl represented by R¹ and/or R² includes a phenyl group substituted or not substituted by at least one of the groups of nitro, halo, optionally substituted alkyl, optionally substituted alkoxy, optionally substituted phenyl, optionally substituted alkylphenyl,
alkoxyphenyl, optionally substituted phenoxy, hydroxyl, cyano, carboxyl, optionally substituted alkoxycarbonyl, optionally substituted phenoxycarbonyl, carbamoyl, sulfo, chlorosulfonyl, optionally substituted alkoxysulfonyl, optionally substituted phenoxysulfonyl, sulfamoyl, mercapto, optionally substituted alkylthio, optionally substituted phenylthio, amino, optionally substituted alkylamino, acylamino, phosphono, alkylphosphonyl, optionally substituted phenylphosphonyl, optionally substituted alkylsulfonyl, phenylsulfonyl, formyl, optionally substituted azo and a group represented by the formula (i) or (ii) : 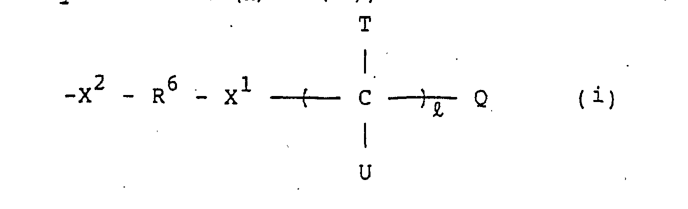 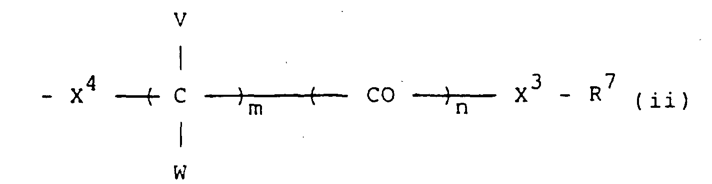 wherein R⁶ is a methylene group or a straight or branched C₂-C₆ alkylene group which may be substituted by hydroxyl group, an C₁₋₆-, preferably an C₁₋₄-alkoxyl group or an C₁₋₆-, preferably are C₁₋₄-alkylcarbonyloxy group, X is a direct linkage or a divalent group of -O-, -S-, 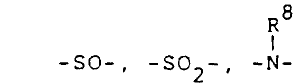 in which R⁸ is a hydrogen atom or an C₁₋₆-, preferably an
C₁₋₄-alkyl group, 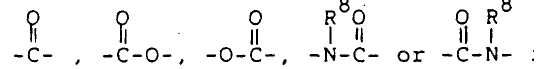 in which
R⁸ is as defined above, X² is -O- or 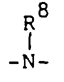 in which R⁸ is
as defined above, T and U are each independently a hydrogen atom or an C₁₋₆-, preferably an C₁₋₄-alkyl group, ℓ is 0 or an integer of 1 to 3, Q is an unsubstituted or substituted 5-, 6- or 7-membered saturated or unsaturated heterocyclic residue which may be condensed with a benzene or heterocyclic ring, R⁷ is an C₁₋₆-, preferably an C₁₋₄-alkyl group, X³ is -O- or -S-, X⁴ is 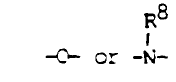 in which R⁸ is as defined above, V and W are each independently a hydrogen atom or an C₁₋₆-, preferably an C₁₋₄-alkyl group, m is an integer of 1 to 3, n is 0 or 1, provided that m+n is at least 2.

In the substituents optionally appended to the phenyl group represented by R¹ and/or R², the alkyl and alkoxy as well as these residues in the alkylamino, alkylthio, alkylsulfonyl, alkoxysulfonyl, alkylphosphonyl, alkoxycarbonyl
and alkylphenyl groups are those having 1 to 4 carbon atoms. Furthermore, they may further be substituted by a halo group such as chloro, bromo, fluoro, etc., a C₁-C₄ alkoxy group, a phenyl group, a C₁-C₄ alkoxyphenyl group, a phenoxy group, a hydroxyl group or a cyano group.

The phenyl and phenyl moiety in the phenoxy, phenoxycarbonyl, phenoxysulfonyl, phenylphosphonyl and phenylthio groups, which are optionally appended to the phenyl group may be substituted or not substituted by a halo group such as chloro, bromo, fluoro, etc. and a nitro group.

The acylamino group includes a formylamino group, a C₂-C₇ acylamino group such as an acetylamino group, a propionylamino group, a butyrylamino group, a valerylamino group, an acryloylamino group and a benzoylamino group.

The unsaturated heterocyclic residue represented by Q includes groups of furyl, thienyl, pyrrolyl, pyridyl, pyranyl, thiazolyl, oxazolyl, pyrazolyl, imidazolyl, thiadiazolyl and s-triazoyl, which are represented by the following formulas, respectively.

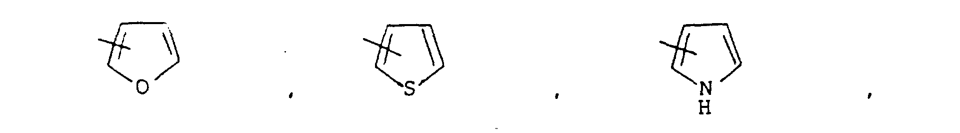

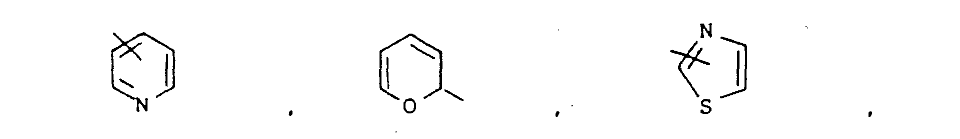

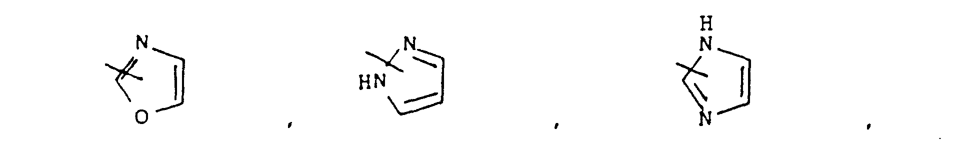

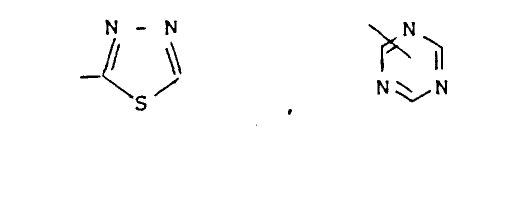

The saturated heterocyclic residue represented by Q includes those represented by the following formulas. 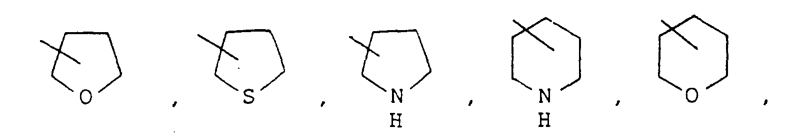 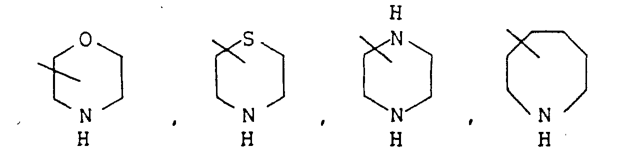 Of these, preferred are groups of tetrahydrofuryl, tetrahydrothienyl, pyrrolidyl, piperidyl, tetrahydropyranyl, piperazinyl, morpholinyl and hexahydroazepinyl, and particularly preferred are tetrahydrofuryl, pyrrolidyl, piperidyl, tetrahydropyranyl and morpholinyl.

The heterocyclic residue in the present invention may be condensed with benzene or heterocyclic ring to form those exemplified below. 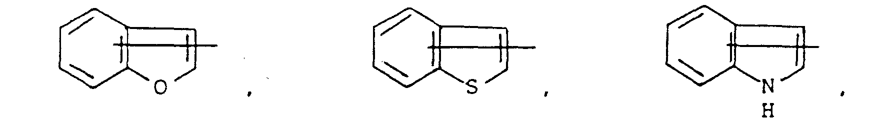 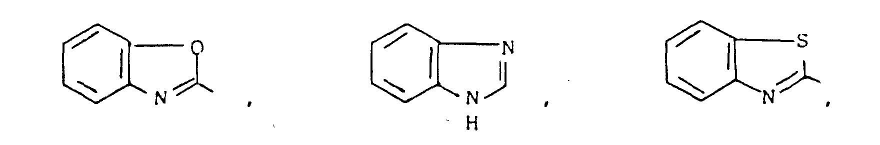 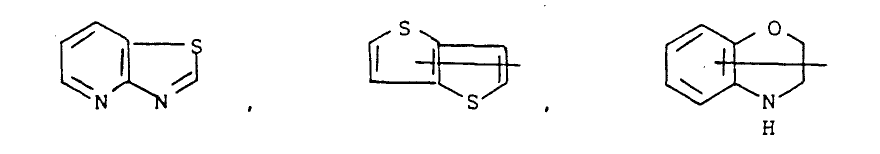 Among them, preferred are those condensed with benzene ring such as benzofuranyl, benzothienyl, indolyl, benzoxazolyl, benzoimidazolyl and benzothiazolyl.

The hetrocyclic residues described above are unsubstituted or substituted by one or two halo groups such as fluoro, chloro and bromo, hydroxyl, C₁-C₄ alkyl, C₁-C₄ alkoxy C₁-C₄ alkylcarbonyl, C₁-C₄ alkoxycarbonyl, cyano, primary, secondary or tertiary amine unsubstituted or substituted by a C₁-C₄ alkyl or keto group.

The benzofuran compounds of the formula (II) mentioned in, for example, JP-A-60-178889 and

EP-A-252406 are prepared by allowing hydroquinone to react with glycolic acid compounds of the following formula (IV), 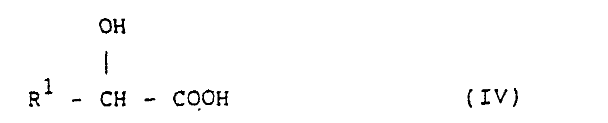 wherein R¹ is as defined above.

The reaction between the hydroquinone and the glycolic acid compounds is carried out preferably in 73% sulfuric acid or a mixture of sulfuric acid and acetic acid [Britrzzcki and Flateau, Ber., 30, 124 (1897)].

The acetonitrile compounds of the formula (III) are prepared by allowing aldehyde compounds of the following formula (V),

R² - CHO (V)

wherein R² is as defined above, to react with metallic cyanide compounds such as potassium cyanide, sodium cyanide and the like in the presence of acid halides represented by the following formula (VI),

L - Hal (VI)

wherein L is as defined above and Hal is a halo group.

The symbol L in the formulas (III) and (VI) is preferably an alkylcarbonyl group such as acetyl, ethylcarbonyl, n- or iso-propylcarbonyl, n-, iso- or tert-butylcarbonyl and the like, a phenylcarbonyl group such as benzoyl, o-, m- or p-methylbenzoyl, o-, m- or p-methoxybenzoyl, o-, m- or p-nitrobenzoyl and the like, an alkoxycarbonyl group such as methoxycarbonyl, ethoxycarbonyl, n- or iso-propoxycarbonyl, n-, iso- or tert-butoxycarbonyl and the like, a phenoxycarbonyl group such as benzeneoxycarbonyl, p-methylbenzeneoxycarbonyl, p-nitrobenzeneoxycarbonyl and the like, an alkanesulfonyl group such as methanesulfonyl, ethanesulfonyl, n- or iso-propanesulfonyl, n- or iso-butanesulfonyl and the like, a phenylsulfonyl group such as benzenesulfonyl, p-methylbenzenesulfonyl, 4-methyl-2-nitrobenzenesulfonyl and the like.

A reaction between the benzofuran compounds (II) and the acetonitrile compounds (III) is carried out stoichiometrically in the presence or absence, preferably in the presence, of a solvent, at a temperature ranging from 30° to 180°C, preferably from 50° to 120°C. The solvents usable for the reaction are hydrocarbons and halogenated hydrocarbons such as benzene, toluene, xylene, mesitylene, mono-, di- and tri-chlorobenzene, bromobenzene, chloronaphthalene, 1,2-dichloroethane, 1,1,1-trichloroethane, 1,1,2,2-tetrachloroethane, trichloroethylene, tetrachloroethylene, dichloromethane, chloroform, carbon tetrachloride, bromoform and low boiling petroleum fractions; nitrated hydrocarbons such as nitrobenzene, nitrotoluene and nitromethane; ketones such as methylisobutyl ketone and sulfolane; alkane carboxylic acids and their anhydrides such as formic acid, acetic acid, propionic acid and acetic anhydride. Among them, preferable are aromatic hydrocarbons and halogenated hydrocarbons such as toluene, monochlorbenzene, dichlorobenzene, 1,2-dichloroethane, 1,1,1-trichloroethane, 1,1,2,2-tetrachloroethane, trichloroethylene, dichloromethane and chloroform.

The reaction between (II) and (III) is carried out in the presence of acid catalysts. Examples of the acid catalysts are unsubstituted or substituted benzenesulfonic acids such as benzenesulfonic acid, p-toluenesulfonic acid and benzene-m-disulfonic acid; alkanesulfonic acids such as methanesulfonic acid; halogenated lower carboxylic acids such as trifluoroacetic acid; Lewis acids such as titanium tetrachloride, aluminum chloride, stannous chloride, stannic chloride, ferrous chloride, ferric chloride, zinc chloride and boron fluoride; mineral acids such as sulfuric acid and hydrochloric acid. Any amount of the acid catalysts may be used, but preferably, 0.5 mole or more, more preferably 0.8 to 2 moles, most preferably 0.9 to 1.5 moles per mole of the benzofuran compounds (II) may be used.

A product produced by the reaction between the benzofuran compounds (II) and the acetonitrile compounds (III) may be isolated, if desired. In the present invention, the product is subjected to a successive oxidation reaction, without the isolation.

The oxidation reaction is carried out preferably in the presence of a solvent at a temperature ranging from 30° to 180°C, preferably from 40° to 100°C. The solvent usable for the reaction between (II) and (III) mentioned above are also used in the oxidation reaction. In order to promote the oxidation reaction, oxidizing agents are preferably used. Examples of the oxidizing agents are chloranil, bromanil, thiosulfate, nitrite, hypochlorite, chlorite, chlorate, perchlorate, persulfate, periodate, perborate, permanganate, vanadate, dichromate, iodine, nitrate, tetrachlorohydroquinone, lead dioxide, manganese dioxide, vanadium pentoxide, nitrobenzene, dicyanodichlorobenzoquinone, hydrogen peroxide and air. They may be used alone or in a mixture thereof, preferably a mixture of two or more. More preferred is a mixture of one nitrite with one selected from chloranil, bromanil and dicyanodichlorobenzoquinone. Particularly preferred is a mixture of sodium nitrite with chloranil. The oxidizing agent is used in a stoichiometric amount or more.

After the oxidation reaction is over, the desired benzodifuranone compounds of the formula (I) are isolated from the reaction mixture in a conventional manner. For instance, a hydrophilic solvent such as lower alkanol is added to the reaction mixture in order to precipitate the desired reaction product, followed by filtration.

The benzodifuranone compounds of the formula (I) are useful as disperse dyes for dyeing or printing hydrophobic fiber materials such as polyester fiber materials, triacetate fiber materials, diacetate fiber materials and polyacetate fiber materials, particularly polyester fiber materials.

Dyeing is conducted in such a manner that the benzodifuranone compounds are finely pulverized in an aqueous medium with the aid of naphthalenesulfonic acid/formaldehyde condensate, lignin sulfonic acid or the like, thereby obtaining a liquid dye dispersion. The liquid dye dispersion may be used as it is or dried with, for example, spray driers to the powdery form. The dyeing is carried out, for example, by a high temperature dyeing method wherein the hydrophobic fiber materials are dipped in an aqueous dye bath, followed by heating to a temperature of 105°C or higher, preferably 110° to 140°C under increased pressures for 30 to 60 minutes. Another dyeing method is a carrier dyeing method wherein the dyeing is carried out in the presence of carriers such as o-phenylphenol, methylnaphthalene and the like at a relatively high temperature, for example, water-boiling temperature. Another is a thermosol method wherein the fiber materials are padded with an aqueous dye dispersion and dry-heated at a temperature of 100°C or higher.

Printing is carried out by mixing the aqueous dye dispersion with suitable stock pastes to obtain a color past, printing the fiber materials with the color pasts and then steaming or thermosol-treating the printed fiber materials.

The process in accordance with the present invention gives the desired benzodifuranone compounds in high purity and high yield using easily available starting materials without difficulty of recovery of the solvent, if used.

The present invention is explained in more detail with reference to the following examples, which are illustrative. In the following examples, parts and % are by weight, unless otherwise defined.

### Example 1

1-Benzoyloxy-1-(p-tetrahydrofurfuryloxyphenyl)-acetonitrile (3.37 parts), 5-hydroxy-2-oxo-3-phenyl-2,3-dihydrobenzofuran (2.26 parts), p-toluenesulfonic acid (2.06 parts) and water (0.22 part) were added to monochlorobenzene (33 parts), and thereafter, the mixture was kept at 80°C for 5 hours. Then, chloranil (2.46 parts) was added to the reaction mixture. The resulting mixture was kept at 80°C for additional 1 hour, therafter cooled to room temperature, and then mixed with methanol (30 parts), and stirred for 1 hour under ice-cooling.

The crystals produced were collected on a filter, washed with methanol (150 parts) and water (30 parts), and then dried to obtain a compound represented by the following formula (1) in high purity and high yield. 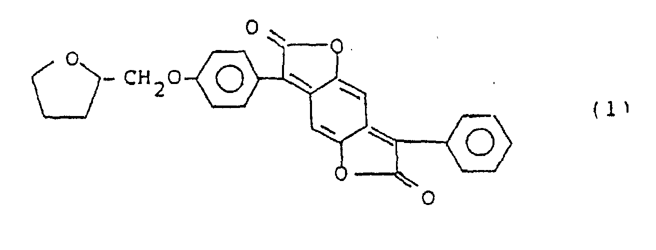 λmax 503 nm (dimethylformamide)

### Example 2

2-Ethoxycarbonyloxy-1-(p-tetrahydrofurfuryl-oxyphenyl)acetonitrile (3.05 parts), 5-hydroxy-2-oxo-3-phenyl-2,3-dihydrobenzofuran (2.26 parts), p-toluenesulfonic acid (2.06 parts) and water (0.22 part) were added to monochlorobenzene (33 parts), and thereafter, the mixture was kept at 75°C for 4 hours. Then, chloranil (2.46 parts) was added to the reaction mixture. The resulting mixture was kept at 75°C for additional 1.5 hours, therafter cooled to room temperature, and then mixed with methanol (35 parts), and stirred for 1 hour under ice-cooling.

The crystals produced were collected on a filter, washed with methanol (150 parts) and water (25 parts), and then dried to obtain a compound represented by the formula (1) in high purity and high yield.

### Example 3

1-Benzoyloxy-1-[p-(3-ethoxypropoxy)phenyl]-acetonitrile (3.40 parts), 5-hydroxy-2-oxo-3-phenyl-2,3-dihydrobenzofuran (2.26 parts), p-toluenesulfonic acid (2.06 parts) and water (0.22 part) were added to monochlorobenzene (30 parts), and thereafter, the mixture was kept at 85°C for 5 hours. Then, chloranil (2.46 parts) was added to the reaction mixture. The resulting mixture was kept at 85°C for additional 1 hour, thereafter cooled to room temperature, and then mixed with methanol (30 parts), and stirred for 1 hour under ice-cooling.

The crystals produced were collected on a filter, washed with methanol (140 parts) and water (35 parts), and then dried to obtain a compound represented by the following formula (2) in high purity and high yield.

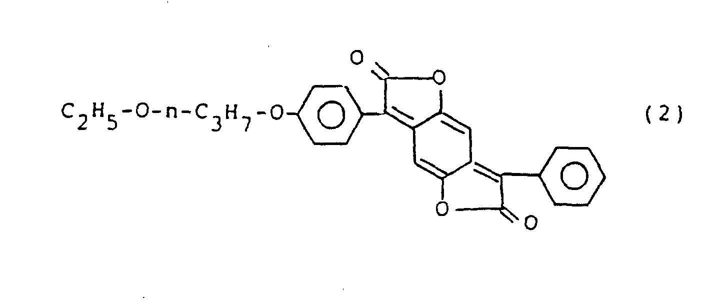

### Example 4

1-Benzoyloxy-1-(p-tetrahydrofurfuryloxyphenyl)-acetonitrile (3.37 parts), 5-hydroxy-2-oxo-3-phenyl-2,3-dihydrobenzofuran (2.26 parts), p-toluenesulfonic acid (2.06 parts) and water (0.22 part) were added to monochlorobenzene (35 parts), and thereafter, the mixture was kept at 80°C for 5 hours. Then, chloranil (0.24 part) and sodium nitrite (0.62 part) were added to the reaction mixture. The resulting mixture was kept at 80°C for additional 2 hours, thereafter cooled to room temperature, and then mixed with methanol (30 parts), and stirred for 1 hour under ice-cooling.

The crystals produced were collected on a filter, washed with methanol (100 parts) and water (30 parts), and then dried to obtain a compound represented by the formula (1) in high purity and high yield.

### Examples 5 to 19

Procedure of Example 1 was repeated, provided that the respective solvents as shown in the Table 1 were used in place of the monochlorobenzene used in Example 1 and the reactions between 1-benzoyloxy-1-(p-tetrahydro-furfuryloxyphenyl)acetonitrile and 5-hydroxy-2-oxo-3-phenyl-2,3-dihyrobenzofuran were carried out at the respective temperatures as shown in the Table 1, thereby obtaining the compound represented by the formula (1) in high purity and high yield.

**Table 1**

| Example No. | Solvent | Amount (parts) | Temperature (°C) |
|---|---|---|---|
| 5 | Benzene | 26.4 | 80 |
| 6 | Toluene | 26.0 | 80 |
| 7 | p-Xylene | 25.8 | 80 |
| 8 | o-Dichlorobenzene | 39.2 | 80 |
| 9 | Bromobenzene | 44.9 | 80 |
| 10 | Chloronaphthalene | 35.8 | 110 |
| 11 | 1,2-Dichloroethane | 37.7 | 80 |
| 12 | 1,1,1-Trichloroethane | 40.1 | 75 |
| 13 | 1,1,2,2-Tetrachloroethane | 47.6 | 80 |
| 14 | Trichloroethylene | 43.9 | 80 |
| 15 | Dichloromethane | 39.8 | 40 |
| 16 | Chloroform | 44.8 | 60 |
| 17 | Nitrobenzene | 35.9 | 90 |
| 18 | Methylisobutyl ketone | 24.0 | 85 |
| 19 | Acetic acid | 31.5 | 80 |

### Examples 20 to 35

Procedure of Example 1 was repeated, provided that the reactions between 1-benzoyloxy-1-(p-tetrahydro-furfuryloxyphenyl)acetonitrile and 5-hydroxy-2-oxo-3-phenyl-2,3-dihydrobenzofuran were carried out using the respective oxidizing agents as shown in the Table 2 in place of the chloranil used in Example 1, thereby obtaining the compound represented by the formula (1) in high purity and high yield.

**Table 2**

| Example No. | Oxidizing agent | Amount (parts) |
|---|---|---|
| 20 | Sodium thiosulfate | 2.48 |
| 21 | Sodium nitrite | 0.69 |
| 22 | 10% Sodium hypochlorite | 7.60 |
| 23 | 10% Potassium hypochlorite | 9.00 |
| 24 | Sodium chlorite | 0.90 |
| 25 | Potassium chlorate | 1.22 |
| 26 | Sodium perchlorate | 1.22 |
| 27 | Ammonium persulfate | 2.28 |
| 28 | Sodium persulfate | 2.38 |
| 29 | Sodium periodate | 2.14 |
| 30 | Potassium permanganate | 1.58 |
| 31 | Nitrobenzene | 1.23 |
| 32 | Dicyanodichlorobenzoquinone | 1.54 |
| 33 | 30% Hydrogen peroxide | 1.13 |
| 34 | Ammonium metavanadate and air | 0.12 |
| 35 | 70% Nitric acid | 0.90 |

### Examples 36 to 46

Procedure of Example 1 was repeated, provided that the respective acid catalysts as shown in the Table 3 were used in place of the p-toluenesulfonic acid used in Example 1 and the reactions between 1-benzoyloxy-1-(p-tetrahydrofurfuryloxyphenyl)acetonitrile and 5-hydroxy-2-oxo-3-phenyl-2,3-dihydrobenzofuran were carried out at the respective temperatures as shown in the Table 3, thereby obtaining the compound represented by the formula (1) in high purity and high yield.

**Table 3**

| Example No. | Acid catalyst | Amount (parts) | Temperature (°C) |
|---|---|---|---|
| 36 | Benzenesulfonic acid | 2.11 | 75 |
| 37 | Benzene-m-disulfonic acid | 2.91 | 65 |
| 38 | Methanesulfonic acid | 1.15 | 95 |
| 39 | Trifluoroacetic acid | 1.37 | 50 |
| 40 | Titanium tetrachloride | 2.27 | 70 |
| 41 | Aluminum chloride | 1.32 | 85 |
| 42 | Stannous chloride | 1.89 | 80 |
| 43 | Ferric chloride | 1.61 | 75 |
| 44 | Boron fluoride (etherate) | 1.14 | 120 |
| 45 | Sulfuric acid | 1.18 | 100 |
| 46 | Hydrochloric acid | 1.06 | 90 |

### Examples 47 to 50

Procedure of Example 1 was repeated, provided that the reactions between 1-benzoyloxy-1-(p-tetrahydro-furfuryloxyphenyl)acetonitrile and 5-hydroxy-2-oxo-3-phenyl-2,3-dihydrobenzofuran were carried out using the respective amount of p-toluenesulfonic acid as shown in the Table 4 in place of the p-toluenesulfonic acid (2.06 parts) used in Example 1, until the compound represented by the formula (1) was obtained in high purity and high yield.

**Table 4**

| Example No. | Amount of p-toluenesulfonic acid (parts) |
|---|---|
| 47 | 1.38 |
| 48 | 1.72 |
| 49 | 2.58 |
| 50 | 3.44 |

### Examples 51 to 55

Procedure of Example 4 was repeated, provided that the reactions between 1-benzoyloxy-1-(p-tetrahydro-furfuryloxyphenyl)acetonitrile and 5-hydroxy-2-oxo-3-phenyl-2,3-dihydrobenzofuran were carried out using the respective amount of chloranil and sodium nitrite as shown in the Table 5 in place of the chloranil (0.24 part) and sodium nitrite (0.62 part) used in Example 4, whereby obtaining the compound represented by the formula (1) in high purity and high yield.

**Table 5**

| Example No. | Amount | |
|---|---|---|
| | Chloranil (parts) | Sodium nitrite (part) |
| 51 | 0.025 | 0.68 |
| 52 | 0.12 | 0.66 |
| 53 | 0.50 | 0.55 |
| 54 | 0.74 | 0.48 |
| 55 | 1.48 | 0.28 |

### Examples 56 to 68

Procedure of Example 1 was repeated, provided that the respective compounds represented by the formula (III) as shown in the Table 6 were used in place of the 1-benzoyloxy-1-(p-tetrahydrofurfuryloxyphenyl)aceto-nitrile (3.37 parts) used in Example 1, until the compound represented by the formula (1) was obtained in high purity and high yield.

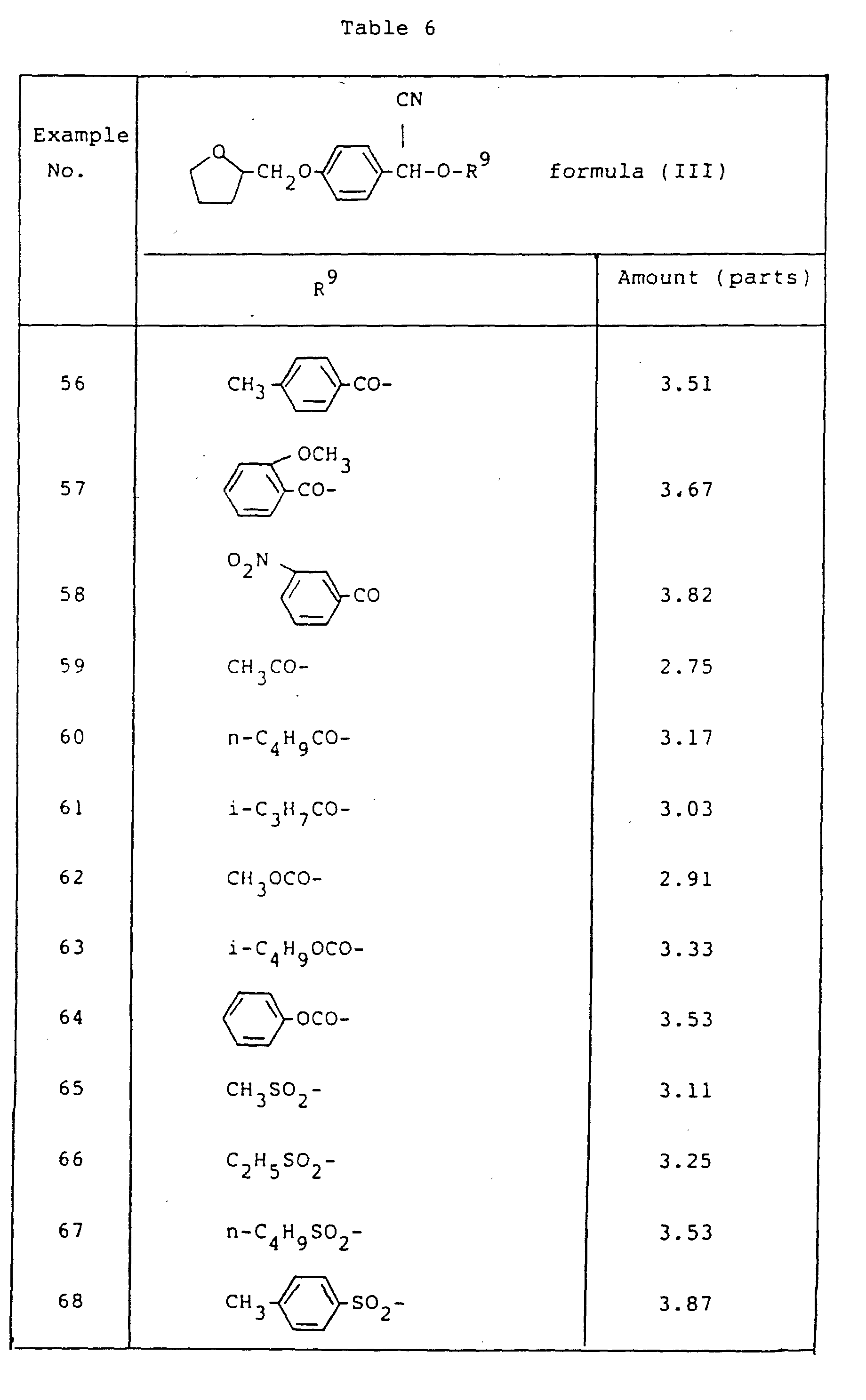

### Examples 69 to 82

In a manner similar to that of Example 1, the respective compounds represented by the formula I-a were obtained in high purity and high yield by allowing the corresponding compounds represented by the formula III-a as shown in the Table 7 to react with 5-hydroxy-2-oxo-3-phenyl-2,3-dihydrobenzofuran.

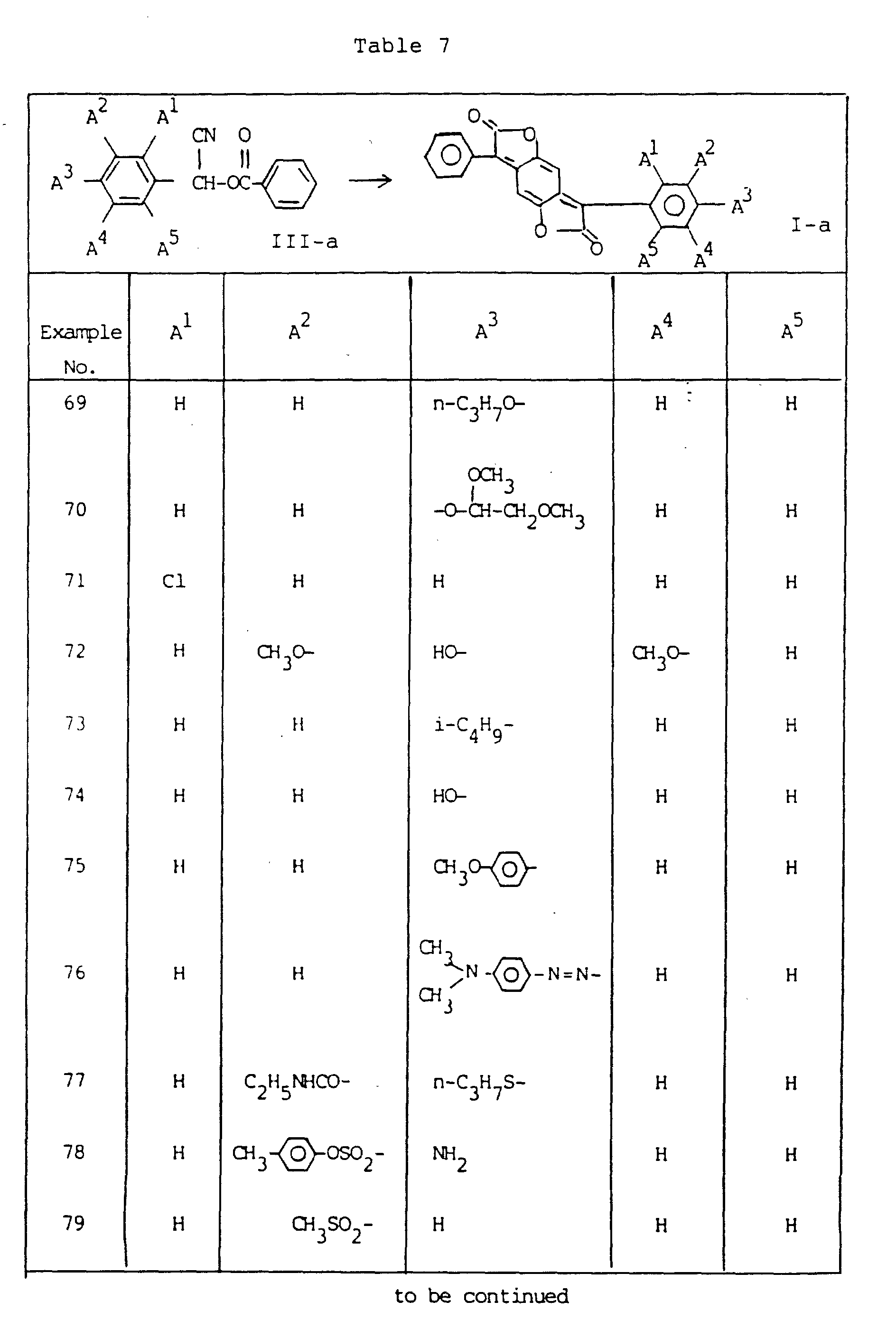

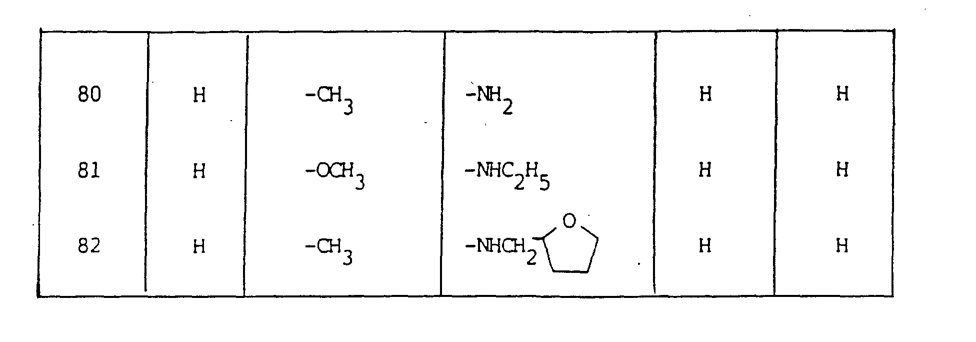

### Examples 83 to 89

In a manner similar to that of Example 1, the respective compounds represented by the formula I-b were obtained in high purity and high yield by allowing the corresponding compounds represented by the formula III-b as shown in the Table 8 to react with 5-hydroxy-2-oxo-3-phenyl-2,3-dihydrobenzofuran.

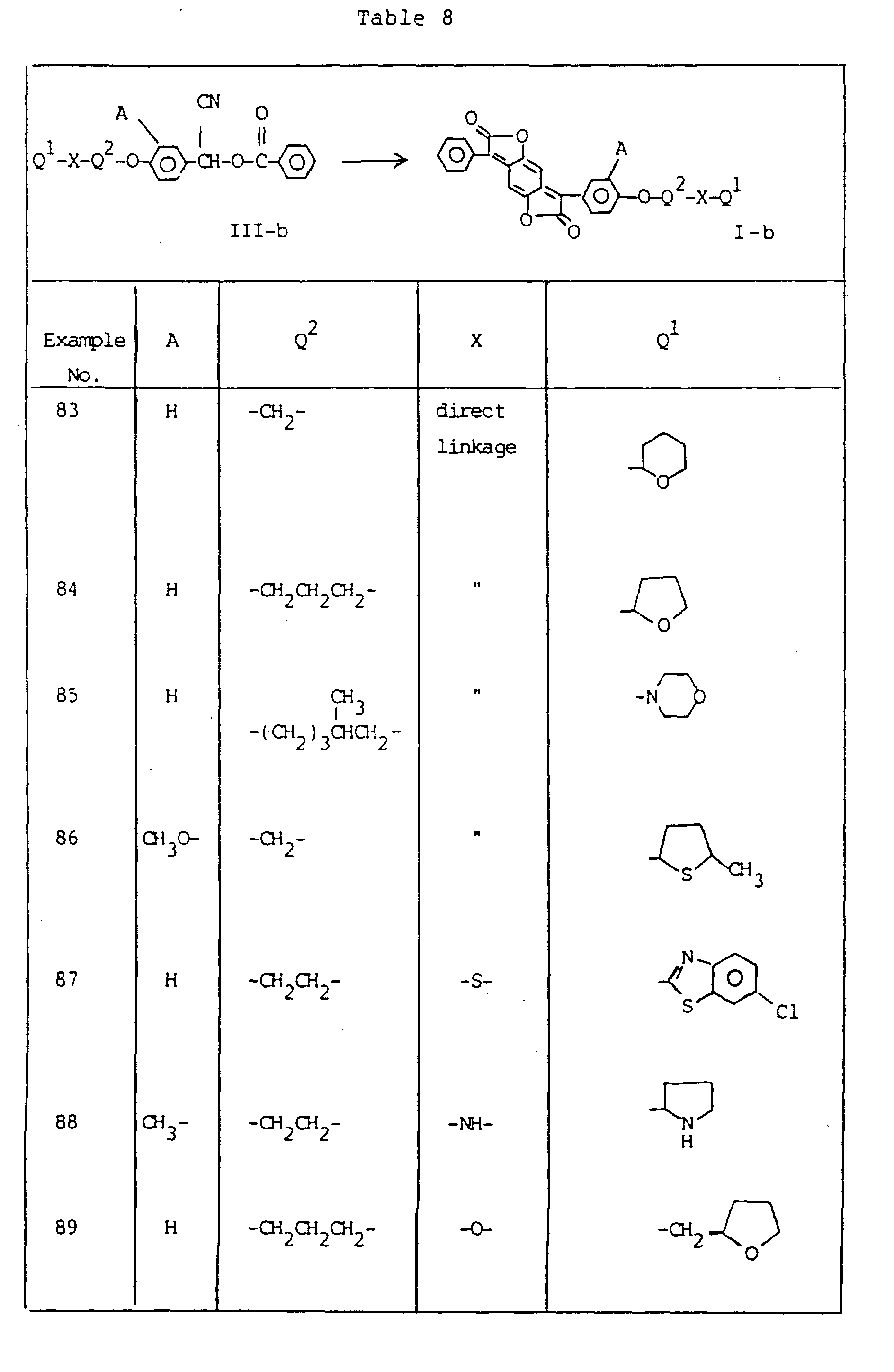

### Example 90

The compound (1.0 part) represented by the formula (1) obtained in Example 1 was finely dispersed in an aqueous medium with the aid of naphthalenesulfonic acid/formaldehyde condensate (3.0 parts). The resulting dye dispersion was dried to powder form.

Polyesther cloth (10 parts, Tetron jersey, a product of Teijin Limited, Japan) was dipped in a dyebath containing the powder obtained (0.6 part), and dyeing was continued for 60 minutes at 130° to 135°C under increased pressures. The dyed cloth was subjected to a reduction-rinsing treatment at 85°C for 10 minutes in a solution of sodium hydroxide (3 parts), hydrosulfite (3 parts) and a betaine amphoteric surfactant (3 parts) in water (3000 parts), washed with water and then dried, thereby obtaining a dyed product having a red color superior in light fastness, sublimation fastness and wet fastness.

### Example 91

A printing paste was prepared by mixing the compound (1.3 part) represented by the formula (1) obtained in Example 1, ligninsulfonic acid (3.7 parts), hot water (35 parts) and a half emulsion paste (60 parts) having the following composition.

| | |
|---|---|
| O/W emulsion | 300 parts |
| Maypro gum 12% paste | 694 parts |
| Sodium chlorate | 4 parts |
| Tartaric acid | 2 parts |
| | 1,000 parts |

Polyesther cloth (Tetron tropical, a product of Teijin Limited, Japan) was printed with the above obtained printing paste, pre-dried and steamed for 7 minutes at 170°C under atmospheric pressure. The printed cloth was subjected to a reduction-rinsing treatment, washing with water and softening and anti-static finishings in this order in a manner similar to that of Example 90. The thus obtained product of a red color was found to have superior light, sublimation and wet fastness properties, particularly washing fastness.

## Claims

1. A process for producing benzodifuranone compounds of the following formula (I), 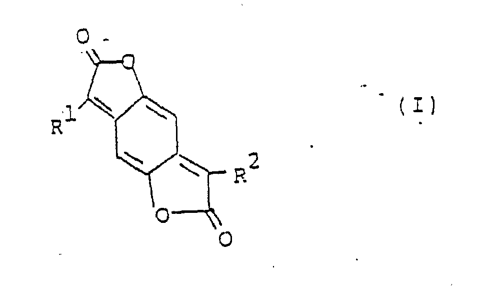 wherein R¹ and R² are each independently a naphthyl group or an unsubstituted or substituted phenyl group, which comprises allowing benzofuran compounds of the following formula (II), 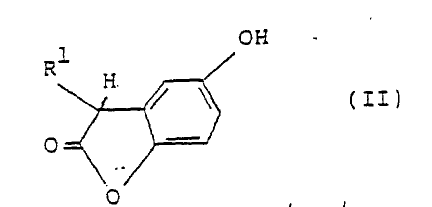 wherein R¹ is as defined above, to react at a temperature of 30° to 180°C in the presence of acid catalysts with
acetonitrile compounds of the following formula (III), 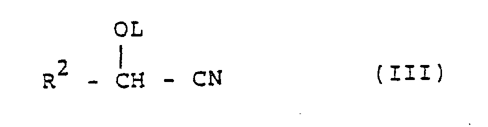 wherein R² is as defined above, and L is -COR³, -CO₂R⁴ or -SO₂R⁵ in which R³, R⁴ and R⁵ are each independently an alkyl or phenyl group, followed by oxidation.

2. The process according to claim 1, wherein the acid catalysts are used in an amount of at least 0.5 mole per mole of the benzofuran compounds.

3. The process according to any one of claims 1 to 2, wherein the reaction between the benzofuran compounds of the formula (II) and the acetonitrile compounds of the formula (III) is carried out in the presence of solvents.

4. The process according to claim 3, wherein the solvents are benzene, toluene, xylene, mesitylene, mono-, di- or tri-chlorobenzene, bromobenzene, chloronaphthalene, 1,2-dichloroethane, 1,1,1-trichloroethane, 1,1,2,2-tetrachloroethane, trichloroethylene, tetrachloroethylene, dichloromethane, chloroform, carbon tetrachloride, bromoform, low boiling petroleum fractions, nitrobenzene, nitrotoluene, nitromethane, methylisobutyl ketone, sulfolane, formic acid, acetic acid, propionic acid or acetic anhydride.

5. The process according to claim 4, wherein the solvents are toluene, monochlorobenzene, dichlorobenzene, 1,2-dichioroethane, 1,1,1-trichloroethane, 1,1,2,2-tetrachloroethane, trichloroethylene, dichloromethane or chloroform.

6. The process according to any one of claims 1 to 5, wherein the reaction between the benzofuran compounds of the formula (II) and the acetonitrile compounds of the formula (III) is carried out at a temperature of 50° to 120°C.

7. The process according to any one of claims 1 to 6, wherein the acetonitrile compounds of the formula (III) are prepared by allowing aldehyde compounds of the following formula (V),
R² - CHO (V)
wherein R² is as defined in claim 1, to react with metallic cyanide compounds in the presence of acid halides of the following formula (VI),
L - Hal (VI)
wherein L is as defined in claim 1, and Hal is a halo group.

8. The process according to claim 1, oxidation is carried out by using oxidizing agents wherein the oxidizing agents are at least one of chloranil, bromanil, thiosulfate, nitrite, hypochlorite, chlorite, chlorate, perchlorate, persulfate, periodate, perborate, permanganate, vanadate, dichromate, iodine,nitrate, tetrachlorohydroquinone, lead dioxide, manganese dioxide, vanadium pentoxide, nitrobenzene, dicyanodichlorobenzoquinone, hydrogen peroxide and air.

9. The process according to claim 8, wherein the oxidizing agents are mixtures of one nitrite with one selected from chloranil, bromanil and dicyanodichlorobenzoquinone.

10. The process according to claim 9, wherein the oxidizing agents are mixtures of chloranil and sodium nitrite.

11. The process according to any one of claims 1 to 10, wherein the oxidation is carried out at a temperature of 30° to 180°C.

## Patentansprüche

1. Verfahren zur Herstellung von Benzdifuranonverbindungen der folgenden Formel (I): 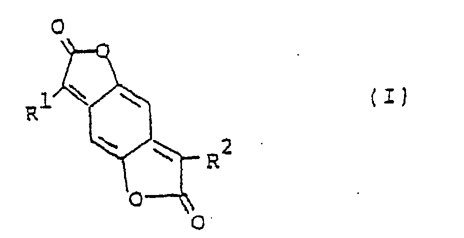 in der R¹ und R² jeweils unabhängig eine Naphthylgruppe oder eine unsubstituierte oder substituierte Phenylgruppe sind, umfassend Reagierenlassen von Benzfuranverbindungen der folgenden Formel (II) 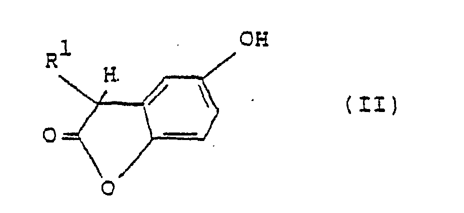 in der R¹ die vorstehend angegebene Bedeutung hat, bei einer Temperatur von 30°C bis 180°C in Gegenwart von sauren Katalysatoren mit Acetonitrilverbindungen der folgenden Formel (III) 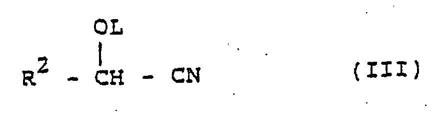 in der R² die vorstehend angegebene Bedeutung hat und L -COR³, -CO₂R⁴ oder -SO₂R⁵ ist, wobei R³, R⁴ und R⁵ jeweils unabhängig ein Alkylrest oder eine Phenylgruppe sind, gefolgt von Oxidation.

2. Verfahren nach Anspruch 1, wobei die sauren Katalysatoren in einer Menge von mindestens 0,5 mol, pro mol der Benzfüranverbindungen, verwendet werden.

3. Verfahren nach einem der Ansprüche 1 bis 2, wobei die Reaktion zwischen den Benzfuranverbindungen der Formel (II) und den Acetonitrilverbindungen der Formel (III) in Gegenwart von Lösungsmitteln durchgeführt wird.

4. Verfahren nach Anspruch 3, wobei die Lösungsmittel Benzol, Toluol, Xylol, Mesitylen, Mono-, Di- oder Trichlorbenzol, Brombenzol, Chlornaphthalin, 1,2-Dichlorethan, 1,1,1-Trichlorethan, 1,1,2,2-Tetrachlorethan, Trichlorethylen, Tetrachlorethylen, Dichlormethan, Chloroform, Tetrachlorkohlenstoff, Bromoform, niedrigsiedende Erdölfraktionen, Nitrobenzol, Nitrotoluol, Nitromethan, Methylisobutylketon, Sulfolan, Ameisensäure, Essigsäure, Propionsäure oder Essigsäureanhydrid sind.

5. Verfahren nach Anspruch 4, wobei die Lösungsmittel Toluol, Monochlorbenzol, Dichlorbenzol, 1,2-Dichlorethan, 1,1,1-Trichlorethan, 1,1,2,2-Tetrachlorethan, Trichlorethylen, Dichlormethan oder Chloroform sind.

6. Verfahren nach einem der Ansprüche 1 bis 5, wobei die Umsetzung zwischen den Benzfuranverbindungen der Formel (II) und den Acetonitrilverbindungen der Formel (III) bei einer Temperatur von 50°C bis 120°C durchgeführt wird.

7. Verfahren nach einem der Ansprüche 1 bis 6, wobei die Acetonitrilverbindungen der Formel (III) durch Reagierenlassen der Aldehydverbindungen der folgenden Formel (V)
R² - CHO (V)
in der R² die in Anspruch 1 angegebene Bedeutung hat, mit Metallcyanidverbindungen in Gegenwart von Säurehalogeniden der folgenden Formel (VI) hergestellt werden
L - Hal (VI)
wobei L die in Anspruch 1 angegebene Bedeutung hat und Hal ein Halogenatom ist.

8. Verfahren nach Anspruch 1, wobei die Oxidation unter Verwendung von Oxidationsmitteln durchgeführt wird, wobei die Oxidationsmittel mindestens eines von Chloranil, Bromanil, Thiosulfat, Nitrit, Hypochlorit, Chlorit, Chlorat, Perchlorat, Persulfat, Perjodat, Perborat, Permanganat, Vanadat, Dichromat, Jod, Nitrat, Tetrachlorhydrochinon, Bleidioxid, Mangandioxid, Vanadiumpentoxid, Nitrobenzol, Dicyanodichlorbenzochinon, Wasserstoffperoxid und Luft sind.

9. Verfahren nach Anspruch 8, wobei die Oxidationsmittel Gemische eines Nitrits mit einer Verbindung, ausgewählt aus Chloranil, Bromanil und Dicyanodichlorbenzochinon, sind.

10. Verfahren nach Anspruch 9, wobei die Oxidationsmittel Gemische von Chloranil und Natriumnitrit sind.

11. Verfahren nach einem der Ansprüche 1 bis 10, wobei die Oxidation bei einer Temperatur von 30°C bis 180°C durchgeführt wird.

## Revendications

1. Procédé de préparation de composés de benzodifurannone répondant à la formule (I) suivante : 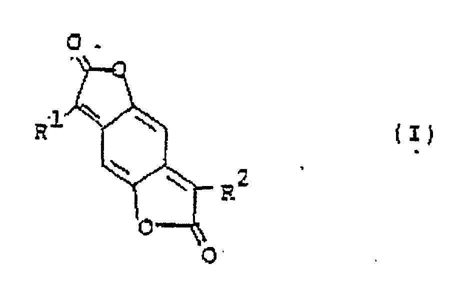 dans laquelle R¹ et R² représentent chacun indépendamment un groupe naphtyle ou un groupe phényle non substitué ou substitué, qui comprend l'opération consistant à amener à réagir des composés de benzofuranne répondant à la formule (II) suivante : 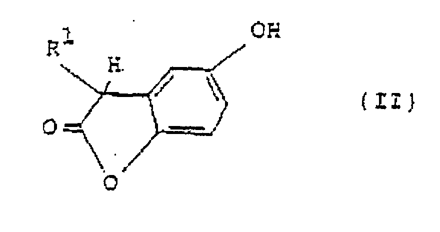 dans laquelle R¹ est tel que défini ci-dessus, à une température de 30 °C à 180 °C, en présence de catalyseurs acides, avec des composés d'acétonitrile répondant à la formule (III) suivante : 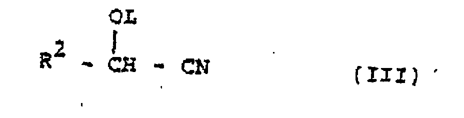 dans laquelle R² est tel que défini ci-dessus, et L représente -COR³, -CO₂R⁴ ou -SO₂R⁵, où R³, R⁴ et R⁵ représentent chacun indépendamment un groupe alkyle ou phényle, puis à les soumettre à une oxydation.

2. Procédé suivant la revendication 1, dans lequel les catalyseurs acides sont utilisés en une quantité d'au moins 0,5 mole par mole des composés de benzofuranne.

3. Procédé suivant l'une quelconque des revendications 1 et 2, dans lequel la réaction entre les composés de benzofuranne de formule (II) et les composés d'acétonitrile de formule (III) est conduite en présence de solvants.

4. Procédé suivant la revendication 3, dans lequel les solvants sont le benzène, le toluène, le xylène, le mésitylène, le mono-, di- ou trichlorobenzène, le bromobenzène, le chloronaphtalène, le 1,2-dichloroéthane, le 1,1,1-trichloroéthane, le 1,1,2,2-tétrachloroéthane, le trichloroéthylène, le tétrachloroéthylène, le dichlorométhane, le chloroforme, le tétrachlorure de carbone, le bromoformyle, des fractions de pétrole à bas point d'ébullition, le nitrobenzène, le nitrotoluène, le nitrométhane, la méthylisobutylcétone, le sulfolane, l'acide formique, l'acide acétique, l'acide propionique ou l'anhydride acétique.

5. Procédé suivant la revendication 4, dans lequel les solvants sont le toluène, le monochlorobenzène, le dichlorobenzène, le 1,2-dichloroéthane, le 1,1,1-trichloroéthane, le 1,1,2,2-tétrachloroéthane, le trichloroéthylène, le dichlorométhane ou le chloroforme.

6. Procédé suivant l'une quelconque des revendications 1 à 5, dans lequel la réaction entre les composés de benzofuranne de formule (II) et les composés d'acétonitrile de formule (III) est conduite à une température de 50 °C à 120 °C.

7. Procédé suivant l'une quelconque des revendications 1 à 6, dans lequel les composés d'acétonitrile de formule (III) sont préparés en amenant à réagir les composés d'aldéhyde répondant à la formule (V) suivante :
R²-CHO (V)
dans laquelle R² est tel que défini dans la revendication 1, avec des composés de cyanure métallique en présence d'halogénures d'acides répondant à la formule (VI) suivante :
L-Hal (VI)
dans laquelle L est tel que défini dans la revendication 1, et Hal est un groupe halo.

8. Procédé suivant la revendication 1, dans lequel l'oxydation est conduite en utilisant des agents oxydants, les agents oxydants étant au moins l'un des composés suivants : le chloranil, le bromanil, un thiosulfate, un nitrite, un hypochlorite, un chlorite, un chlorate, un perchlorate, un persulfate, un periodate, un perborate, un permanganate, un vanadate, un dichromate, l'iode, un nitrate, la tétrachlorohydroquinone, le dioxyde de plomb, le dioxyde de manganèse, le pentoxyde de vanadium, le nitrobenzène, la dicyanodichlorobenzoquinone, le peroxyde d'hydrogène et l'air.

9. Procédé suivant la revendication 8, dans lequel les agents oxydants sont des mélanges d'un nitrite et d'un composés consistant en le chloranil, le bromanil et la dicyanodichlorobenzoquinone.

10. Procédé suivant la revendication 9, dans lequel les agents oxydants sont des mélanges de chloranil et de nitrite de sodium.

11. Procédé suivant l'une quelconque des revendications 1 à 10, dans lequel l'oxydation est conduite à une température de 30 °C à 180 °C.
